# FASCICULE DE BREVET EUROPEEN

(11) **EP 1 409 692 B1**
(45) Date de publication et mention de la délivrance du brevet: **17.09.2008**
(21) Numéro de dépôt: 02770043.4
(22) Date de dépôt: 19.07.2002
(51) Int. Cl.: C12N 15/62, C07K 14/135, C07K 16/08, A61K 39/155

(54) **NOUVEAUX PEPTIDES DERIVES DE LA PROTEINE G DU VRS ET LEUR UTILISATION DANS UN VACCIN**
PEPTIDE AUS DEM G-PROTEIN DES RESPIRATORISCHEN SYNZYTIALVIRUS UND DEREN VERWENDUNG IN EINEM IMPFSTOFF
NOVEL PEPTIDES OF THE RESPIRATORY SYNCYTIAL VIRUS (RSV) G PROTEIN AND THEIR USE IN A VACCINE

(30) Priorité: 20.07.2001 FR 0109731
(43) Date de publication de la demande: 21.04.2004
(73) Titulaire: PIERRE FABRE MEDICAMENT, 92100 Boulogne-Billancourt (FR)
(72) Inventeur: CORVAIA, Nathalie, F-74160 St Julien Genevois (FR); NGUYEN NGOC, Thien, F-31180 Rouffiac-Tolosan (FR); BECK, Alain, F-74160 Collonges sous Salève (FR); PLOTNICKY GILQUIN, Hélène, F-74270 Chaumont (FR)
(74) Mandataire: Ahner, Francis
(86) Numéro de dépôt international: PCT/FR2002/002599
(87) Numéro de publication internationale: WO 2003/010317

(56) Documents cités:
- WO-A-01/21203
- WO-A-95/27787
- SIMARD C. ET AL.: "Subgroup specific protection of mice from respiratory syncytial virus infection with peptides encompassing the amino acid region 174-187 from the G glycoprotein: the role of cysteinyl residues in protection" VACCINE, vol. 15, no. 4, 1997, pages 423-432, XP002194801
- VON HUNOLSTEIN C ET AL.: "The adjuvant effect of synthetic oligodeoxynucleotide containing CpG motif converts the anti-Haemophilus type b glycoconjugates into efficient anti-polysaccharide and anti-carrier polyvalent vaccines" VACCINE, vol. 19, no. 23/24, 30 avril 2001 (2001-04-30), pages 3058-3066, XP002194802

## Description

La présente invention se rapporte au virus respiratoire syncytial, et plus particulièrement à l'identification de nouveaux antigènes, utiles notamment pour le traitement thérapeutique et prophylactique des affections provoquées par ce virus.

Le Virus Respiratoire Syncytial (VRS) est classé dans la famille des Paramyxoviridae, genre pneumovirus comportant un génome ARN non segmenté, de polarité négative, codant pour 11 protéines spécifiques.

Le VRS est l'un des agents étiologiques les plus fréquemment rencontrés chez le nourrisson et chez les personnes âgées. Les bronchiolites sont souvent graves chez l'enfant et nécessitent l'hospitalisation. Actuellement il n'existe pas de moyens de prévention contre la maladie due au VRS. La première infection au VRS ne prévient pas contre la suivante. Le traitement des cas graves par antibiothérapie (Ribavirine) et/ou associé avec l'immunothérapie (immunoglobulines humains) ne peut pas atténuer l'aggravation de la maladie. Un anticorps monoclonal humanisé dirigé contre la protéine F du VRS dénommé palivizumab (Synagis TM) a également été développé. Toutefois, ce type de traitement reste encore très coûteux. Dans les années 60, les tentatives d'immunisation des enfants avec un vaccin VRS inactivé à la formaline (FI-VRS) avaient eu pour conséquence l'aggravation de la maladie au lieu de conférer une protection contre l'infection naturelle au VRS. Cette aggravation de la maladie a été caractérisée par une augmentation des neutrophiles polymorphonucléaires, des lymphocytes et des éosinophiles dans le sang et les poumons (Kim et al., Pediatric Res., 10:75-78, 1976). Il a aussi été démontré chez la souris que FI-VRS induisait une réponse immune de type Th2 (T-helper 2), se traduisant notamment par une production importante d'IL-4, d'IL-5, d'IL-10 et d'IL-13, des cytokines Th2. Des études récentes ont permis de corréler cette immunopathologie observée suite à l'administration de FI-VRS avec une région précisément déterminée, un épitope CD4+ de séquence 185-193 de la protéine G (ICKRIPNKK, SEQ ID N° 10), qui s'avère primordiale pour obtenir une réponse en cytokines Th2 chez les souris (Varga et al., J. Immunol., 165:6487-6495, 2000).

La demande WO 87/04185 a proposé d'utiliser des protéines structurales du VRS en vue d'un vaccin, comme les protéines d'enveloppe appelées protéine F (protéine de fusion) ou protéine G (protéine d'attachement), une glycoprotéine de 22 Kd, une protéine de 9,5 Kd, ou la protéine majeure de capside (protéine N).

La demande WO 89/02935 décrit les propriétés de protection de la protéine F entière du VRS, éventuellement modifiée sous forme monomérique ou déglycosylée.

Dans la demande WO 95/27787, il a été montré que la protéine G du VRS peut être utile dans la préparation de produits destinés au traitement et/ou à la prévention d'affections provoquées par le VRS, sous-groupe A ou B.

Des peptides structurellement homologues à la séquence 149-197 de la protéine G et dans laquelle aucun oligosaccharide n'est lié à une sérine, thréonine ou asparagine sont décrits dans la demande WO 97/46581.

La demande WO 99/03987 quant à elle décrit des fragments de la protéine G du VRS, contenant des épitopes spécifiques, utilisés dans un vaccin contre l'infection au VRS.

Toutefois, aucune de ces demandes n'a résolu le problème de la mise au point d'antigènes du VRS permettant d'obtenir à la fois une réponse immune suffisante et protectrice, une protection croisée VRS A et B et présentant le moins de risque possible d'immunopathologies associées à la production de cytokines de type Th2.

De plus, pour des vaccins destinés aux nouveaux nés, il est aussi souhaitable que l'antigène utilisé présente une hypersensibilité immédiate (ou HSI) négative. L'hypersensibilité immédiate ou anaphylactique à IgE, de type I selon la classification de Gell et Coombs, regroupe les manifestations cliniques observées lors de certaines affections respiratoires, oculaires, cutanées, digestives, .... On peut corréler une réponse HSI positive à une réponse de type Th2 avec production d'IL-5 et d'IgE ; ainsi, de manière à réduire le risque de pathologies associées à une vaccination contre le VRS, il est souhaitable de ne pas induire par le biais de la molécule vaccinale une réponse de type Th2.

Il a aussi été constaté par les inventeurs que la production de peptides dérivés de la protéine G posaient de multiples problèmes, notamment au niveau de la formation des ponts disulfures, qui doivent se trouver dans la même configuration que celle de la protéine G native. En effet, l'appariement natif entre les ponts disulfures doit être respecté tout en conservant un bon rendement.

Ainsi, l'objet de la présente invention est d'obtenir de nouveaux peptides dérivés de la protéine G répondant aux problèmes ci-dessus mentionnés, faciles à produire industriellement et permettant d'obtenir une réponse immune tout comme une protection suffisante, une protection croisée VRS A et B, et le moins de risque possible d'immunopathologies et présentant notamment une HSI négative.

De manière surprenante, il a été mis en évidence qu'un peptide immunogène dérivé de la protéine G du VRS du sous-groupe A ou B comprenant au moins :
- un premier peptide dérivé de la protéine G du VRS du sous-groupe A ou B comprenant au moins en position 173, 176, 182 et 186 une cystéine, et dont l'extrémité C-terminale comprend au plus l'acide aminé en position 192 ; et
- un deuxième peptide dérivé d'une protéine du VRS du sous-groupe A ou B, ledit deuxième peptide étant situé en aval dudit premier peptide, de manière à ce que le peptide immunogène produit présente un pont disulfure reliant les résidus 173 et 186 et un deuxième pont disulfure reliant les résidus 176 et 182 répond aux problèmes mentionnés ci-dessus.

Ainsi, la présente invention a pour objet un peptide immunogène dérivé de la protéine G du VRS du sous-groupe A ou B comprenant au moins :
- un premier peptide dérivé de la protéine G du VRS du sous-groupe A ou B comprenant au moins en position 173, 176, 182 et 186 une cystéine, et dont l'extrémité C-terminale comprend au plus l'acide aminé en position 192 ; et
- un deuxième peptide dérivé d'une protéine du VRS du sous-groupe A ou B, ledit deuxième peptide étant situé en aval dudit premier peptide,
de manière à ce que le peptide immunogène produit présente un pont disulfure reliant les résidus 173 et 186 et un deuxième pont disulfure reliant les résidus 176 et 182, caractérisé en ce que ledit peptide immunogène présente la séquence SEQ ID N°1, SEQ ID N°2 ou SEQ ID N°3.

Par le terme « peptide immunogène », on entend désigner tout peptide qui, lorsqu'il est associé à un porteur ou un adjuvant, est capable de générer ou accroître une réponse immunitaire dirigée contre le VRS. Préférentiellement, ce peptide immunogène permet également d'obtenir une protection croisée VRS A et B.

Il doit être entendu que, lorsque ledit deuxième peptide est choisi parmi les peptides dérivés de la protéine G du VRS, cedit deuxième peptide n'est pas un peptide naturellement contigu en aval dudit premier peptide dans la séquence de ladite protéine G, ceci pour éviter de retomber sur un peptide immunogène dont la séquence serait naturellement incluse dans la séquence sauvage de ladite protéine G, ou dans celle de l'un de ses variants naturelles. En effet ces séquences, déjà décrites dans les documents de l'art antérieur cités ci-avant ne permettent pas d'obtenir à la fois la formation et la configuration des ponts disulfures escomptés (voir ci-après) et une HSI négative.

Dans la présente invention, on entendra désigner par le terme « peptide » également les polypeptides.

Par le terme "situé en aval dudit premier peptide", il faut comprendre que le deuxième peptide est situé en position 3' par rapport au premier peptide.

Par peptide "comprenant au moins en position 173, 176, 182 et 186 une cystéine, et dont l'extrémité C-terminale comprend au plus l'acide aminé en position 192", on entend désigner tout peptide présentant au moins 4 cystéines dans la même configuration que la protéine G native. Les numéros de position font référence à la protéine G native du VRS et ne signifient pas que le premier peptide selon l'invention comprend forcément tous les 192 acides aminés de la protéine native, mais que ce peptide est un peptide de séquence n-m, avec n = I-172 et m = 187-192.

Par la protéine G du VRS du sous-groupe A ou B on entend désigner la protéine d'enveloppe du VRS A ou B.

Il est décrit que le peptide selon l'invention peut être synthétisé en un seul bloc, i.e. est en fait un seul peptide que l'on peut considérer comme l'assemblage d'un premier et deuxième peptide tel que défini ci-dessus.

Ces deux peptides peuvent également être couplés. Le couplage est de préférence un couplage covalent, qui peut être réalisé par la voie chimique ou par des techniques d'ADN recombinant.

Il est décrit que peptide peut notamment être obtenu par synthèse chimique peptidique classique, de préférence sans étapes de glycosylation, connue de l'homme du métier ou par voie recombinante, de préférence sans glycosylation.

Les méthodes de préparation des peptides recombinants glycosylés ou de préférence non glycosylés sont aujourd'hui bien connues de l'homme de l'art et ne seront pas développées dans la présente description. Parmi les cellules utilisables pour la production de ces protéines recombinantes, on peut notamment citer les cellules bactériennes (Olins P.O. et Lee S.C., Curr. Op. Biotechnology, 4:520-525, 1993), et plus particulièrement E. coli.

Il est décrit que l'extrémité C-terminale dudit premier peptide comprend au plus l'acide aminé en position 190.

Il est décrit également que ledit premier peptide présente une séquence choisie parmi la séquence de la protéine G du VRS de sous groupe A ou B 130-190, 130-192, 140-190, 140-192, 145-190, 145-192, 148-190, 148-192, 130-188, 140-188, 145-188 ou 148-188, et préférentiellement la séquence 140-190.

Il est décrit encore que ledit deuxième peptide est constitué d'une chaîne d'au moins 5 acides aminés, de préférence 6, 7, 8, 9 et 10 acides aminés.

En effet, comme cela a été mis en évidence dans les exemples ci-après, il est nécessaire que le fragment du peptide immunogène selon l'invention contigu à la position 186 dudit premier peptide comprennent au moins plus de 4 acides aminés pour permettre d'avoir la formation et la configuration des ponts disulfures escomptées.

Il est décrit également que, ledit deuxième peptide contient au moins un épitope B du VRS A ou B. Ce peptide peut notamment être dérivé de

Il est décrit également que, ledit deuxième peptide est choisi parmi les fragments de la protéine G du VRS comprenant au moins le fragment 144-158 de la protéine G du VRS, l'extrémité C- terminale dudit deuxième peptide comprenant au plus l'acide aminé en position 172.

Ainsi, ledit deuxième peptide peut présenter une séquence choisie parmi la séquence 144-158, 144-159 de la protéine G du VRS et parmi les peptides neutralisants décrits de la protéine F (décrit par Trudel et al., J. Gen. Virol., 68:2273-2280, 1987 ; Trudel et al., Can. J. Microbiol. 33:933-938, 1987 ; Lopez et al., J. Virol., 64:927-930, 1990 et Scopes et al., J. Gen. Virol., 71:53-59, 1990) tels que les peptides de séquence 221-237, 274-287, 262-268 et 483-488 de la protéine F du VRS.

Il est décrit que, ledit deuxième peptide présente la séquence 144-158 ou 144-159 de la protéine G du VRS.

Il est décrit que, ledit peptide immunogène dérivé de la protéine G du VRS du sous-groupe A ou B présente un HSI négatif.

Comme exemple de peptides, on peut citer les peptides immunogènes constitués d'un premier peptide présentant une séquence choisie parmi la séquence de la protéine G du VRS de sous groupe A ou B 140-190 ou 140-192 et d'un deuxième peptide présentant une séquence choisie parmi la séquence 144-158 ou 144-159 de la protéine G du VRS.

Ainsi, la présente invention a pour objet les peptides de séquences SEQ ID N° 1 (dénommé G20a), SEQ ID N° 2 (dénommé G20aP) et SEQ ID N° 3 (dénommé G23a).

L'invention concerne également les séquences d'acide nucléique codant pour un peptide selon l'invention.

L'invention a encore pour objet une composition pharmaceutique caractérisée en ce qu'elle comprend dans un milieu pharmaceutiquement acceptable au moins un peptide selon l'invention ou une séquence d'acide nucléique codant pour un tel peptide.

Ces compositions selon l'invention peuvent en outre contenir au moins une protéine porteuse et/ou un adjuvant.

Il est décrit que la protéine porteuse peut être avantageusement choisie parmi la protéine TT (tetanus toxoïde), la protéine DT (toxoïde diphtérique), la protéine de liaison à la sérumalbumine humaine du Streptocoque et ses fragments, la toxine cholérique (CT) ou sa sous unité B (CTB), l'entérotoxine d'E. coli (LT) ou sa sous unité B (LTB) et les extraits de protéines membranaires bactériennes telles que les OMPC de *Neisseria meningitidis* (Vella et al., Infect. Immun., 60:4977-4983, 1992), TraT *d'Escherichia coli* (Croft et al., J. Immunol., 146:793-798, 1991) ou PorB de *Neisseria meningitidis* (Fusco et al., J. Infect. Dis., 175:364-372, 1997) ou toute autre protéine présentant un épitope Th.

Il est décrit que l'une des protéines porteuses peut consister en une OmpA d'une bactérie du genre Klebsiella, protéine majeure de la membrane externe baptisée P40, présentant une activité de protéine porteuse, par voie systémique, pour des antigènes sous-unitaires peptidiques (WO 95/27787 et WO 96/14415 ; Haeuw et al., Eur. J. Biochem., 255:446-454, 1998 ; Plotnicky-Gilquin et al., J. Virol., 73:5637-5645, 1999).

La séquence d'acide aminé de la protéine P40 est par exemple identifiée dans la liste des séquences du document WO 99/49892 par la séquence SEQ ID N° 1.

Un porteur particulièrement préféré consiste en des dérivés atoxiques de la protéine DT (toxoïde diphtérique), dans lesquels au moins un résidu cystéine a été supprimé. Comme exemple de tel porteur, on peut citer les protéines de séquences SEQ ID N° 4 (dénommé DTa), SEQ ID N° 5 (dénommé DTb), et SEQ ID N° 6 (dénommé DTaDTb).

Selon la présente invention, l'adjuvant peut notamment être choisi parmi le MPL-A (« MonoPhosphoryl Lipid A »), le MF-59^{®}, le Quil-A^{®} (adjuvant dérivé de saponine), l'ISCOM (« ImmunoStimulating COMplex »), le Diméthyl Dioctadécyl Ammonium sous forme de bromure (DDAB) ou de chlorure (DDAC), l'alum (hydroxyde d'aluminium), l'adjuphos, les CpG (oligodésoxynucléotides contenant un motif spécifique centré sur un dinucléotide CpG), la Leif (facteur d'initiation, d'élongation de Leishmania, antigène protéique dérivé de Leishmania capable de stimuler les cellules PBMC et présentatrices d'antigène, et de produire une réaction cytokine de type Th-1), la CT (Toxine Cholérique), la LT (« heat Labil Toxin ») et les versions détoxifiées de la CT ou la LT

Le peptide selon l'invention peut être associé, notamment par mélange ou par couplage, à la protéine porteuse.

Il est décrit que le couplage est de préférence un couplage covalent, qui peut être réalisé par la voie chimique ou par des techniques d'ADN recombinant

Il est décrit un mode de réalisation particulier dans lequel on introduit un ou plusieurs éléments de liaison dans le peptide selon l'invention et/ou dans ledit porteur pour faciliter le couplage chimique, ledit élément de liaison introduit pouvant être un acide aminé.

Ainsi, il est possible d'introduire un ou plusieurs éléments de liaison, notamment .des acides aminés pour faciliter les réactions de couplage entre le peptide selon l'invention et le porteur. Le couplage covalent entre le peptide selon l'invention et ledit porteur peut être réalisé à l'extrémité N- ou C- terminale dudit peptide. Les réactifs bifonctionnels permettant ce couplage seront déterminés en fonction de l'extrémité dudit peptide choisi pour effectuer le couplage et de la nature dudit porteur à coupler.

Le couplage entre le peptide selon l'invention et ledit porteur peut être réalisé par recombinaison génétique, lorsque ledit porteur est de nature peptidique.

Les conjugués issus d'un couplage entre le peptide selon l'invention et ledit porteur peuvent être préparés par recombinaison génétique. La protéine chimérique ou hybride (le conjugué) peut être produite par des techniques d'ADN recombinant par insertion ou addition à la séquence d'ADN codant pour ledit peptide selon l'invention, d'une séquence codant pour ledit porteur de nature protéique.

Les procédés de synthèse des molécules hybrides englobent les méthodes utilisées en génie génétique pour construire des polynucléotides hybrides codant pour les séquences polypeptidiques recherchées. On pourra, par exemple, se référer avantageusement à la technique d'obtention de gènes codant pour des protéines de fusion décrites par D.V. Goeddel (Gene expression technology, Methods in Enzymology, vol. 185, 3-187, 1990).

Ainsi l'invention a pour objet des peptides selon l'invention comprenant aussi un dérivé de la protéine DT (toxoïde diphtérique), dans lesquels au moins un résidu cystéine a été supprimé caractérisé par une des séquences SEQ ID N° 7 (dénommé G20a-DTa), SEQ ID N° 8 (dénommé G20a-DTb) et SEQ ID N° 9 (dénommé G20a-DTaDTb), ainsi que les acides nucléiques codant pour les polypeptides de séquences SEQ ID N° 7, SEQ ID N° 8 et SEQ ID N° 9.

Il est décrit que le peptide selon l'invention peut être conjugué à la protéine porteuse par une protéine de liaison ; cette protéine de liaison peut notamment être choisie parmi un récepteur de l'albumine sérique de mammifère et les récepteurs présents à la surface des cellules mucosales.

L'invention a également pour objet la composition selon l'invention, caractérisée en ce que ladite composition pharmaceutique comprend en outre au moins un deuxième antigène, immunogène ou haptène du VRS et/ou un antigène, immunogène ou haptène dérivé de micro-organisme responsable de pathologies des voies aériennes choisi parmi les parainfluenza virus (VIP 1, 2, 3 et 4), l'influenza virus (A et B), les hantavirus, les streptocoques, les pneumocoques, haemophilus influenza type b, les rhinovirus, les coronavirus et les méningocoques.

Par « immunogène, antigène ou haptène », on entend désigner en particulier tout composé exprimé par un agent infectieux, ou un de leurs analogues structuraux, qui seul ou en association avec un adjuvant ou porteur est capable d'induire une réponse immunitaire spécifique dudit agent infectieux.

On entend également désigner par "immunogène, antigène ou haptène" dans la présente description un composé présentant une analogie structurale avec ledit antigène ou haptène capable d'induire une réponse immunologique dirigée contre ledit antigène ou haptène dans un organisme préalablement immunisé avec ledit composé analogue.

Il est décrit ledit deuxième antigène du VRS peut comprendre au moins un fragment de la protéine G du virus respiratoire syncytial, ledit fragment comprenant un épitope T ou étant uniquement composé dudit épitope T.

Il est décrit également que ledit deuxième antigène du VRS comprend au moins un fragment de la protéine F du virus respiratoire syncytial, ledit fragment comprenant un épitope T ou étant uniquement composé dudit épitope T.

Au sens de la présente invention, le milieu pharmaceutiquement acceptable est le milieu dans lequel les composés de l'invention sont administrés, préférentiellement un milieu injectable chez l'homme. Il peut être constitué d'eau, d'une solution aqueuse saline ou d'une solution aqueuse à base de dextrose et/ou de glycérol.

L'invention comprend également une composition selon l'invention, caractérisé en ce que ladite composition pharmaceutique est véhiculée sous une forme permettant d'améliorer sa stabilité et/ou son immunogénicité ; ainsi, elle peut être véhiculée sous forme de liposomes, virosomes, nanosphères, microsphères ou microcapsules.

L'invention a également pour objet des anticorps, monoclonaux ou polyclonaux, dirigés contre les peptides selon l'invention.

Les anticorps monoclonaux sont, de préférence, humanisés et peuvent être produits par la voie recombinante. Ils peuvent être aussi obtenus par la méthode de librairie de phages.

Il est décrit que l'anticorps monoclonal, polyclonal ou l'un de leurs fragments, est caractérisé en ce qu'il est capable de se fixer spécifiquement sur un épitope ou déterminant des peptides non glycosylés selon l'invention.

Les anticorps monoclonaux pourront avantageusement être préparés à partir d'hybridomes selon la technique décrite par Kohler et Milstein en 1975 (Nature, 256:495-497, 1975).

Les anticorps polyclonaux pourront être préparés, par exemple, par immunisation d'un animal, en particulier une souris ou un lapin, avec le peptide selon l'invention associé à un adjuvant de la réponse immunitaire, puis purification des anticorps spécifiques contenus dans le sérum des animaux immunisés sur une colonne d'affinité sur laquelle a préalablement été fixé ledit peptide ayant servi d'antigène.

Il décrit également tout fragment d'anticorps monoclonal de l'invention capable de se fixer sur un épitope du peptide selon l'invention sur lequel se fixe l'anticorps monoclonal ou polyclonal dont ledit fragment est issu. Des exemples de tels fragments incluent en particulier des anticorps monoclonaux simple chaîne ou des fragments monovalents Fab ou Fab' et des fragments divalents tels que F(ab')2, qui possèdent la même spécificité de fixation que l'anticorps monoclonal ou polyclonal dont ils sont issus. Un tel fragment pourra également être un fragment Fv simple chaîne produit par des méthodes connues de l'homme de l'art et telles que décrites par exemple par Skerra et al. (Science, 240:1038-1041, 1988) et King et al. (Biochemical J., 290:723-729, 1991).

Il est décrit que, des fragments d'anticorps monoclonaux ou polyclonaux peuvent être obtenus à partir des anticorps monoclonaux ou polyclonaux tels que décrits précédemment par des méthodes telles que la digestion par des enzymes, comme la pepsine ou la papaïne et/ou par clivage des ponts disulfures par réduction chimique. D'une autre manière, les fragments d'anticorps monoclonaux ou polyclonaux peuvent être synthétisés par des synthétiseurs automatiques de peptides tels que ceux fournis par la société Applied Biosystems, etc., ou peuvent être préparés manuellement en utilisant des techniques connues de l'homme de l'art et telles que décrites par exemple par Geysen et al. (J. Immunol. Methods, 102:259-274, 1978).

En général, pour la préparation d'anticorps monoclonaux, polyclonaux ou leurs fragments, on pourra se référer aux techniques qui sont en particulier décrites dans le manuel « Antibodies » (Harlow et al., Antibodies: A Laboratory Manual, Cold Spring Harbor Publications pp. 726, 1988) ou à la technique de préparation à partir d'hybridomes décrite par Kohler et Milstein en 1975.

Les anticorps monoclonaux humanisés selon l'invention ou leurs fragments peuvent être préparés par des techniques connues de l'homme de l'art (Carter et al., PNAS 89:4285-4289, 1992 ; Mountain et al., Biotechnol. Genet. Eng. Rev., 10:1-142, 1992).

De tels anticorps monoclonaux humanisés selon l'invention sont cités ici pour leur utilisation dans des méthodes thérapeutiques.

Les anticorps de l'invention, ou leurs fragments pourront également être marqués par un marquage de type enzymatique, fluorescent ou radioactif.

Les anticorps monoclonaux marqués selon l'invention ou leurs fragments incluent par exemple des anticorps dits immunoconjugués qui peuvent être conjugués par exemple avec des enzymes telles que la péroxydase, la phosphatase alkaline, la (β-D-galactosidase, la glucose oxydase, la glucose amylase, l'anhydrase carbonique, l'acétylcholinestérase, le lysozyme, la malate déhydrogénase ou la glucose-6 phosphate déhydrogénase ou par une molécule comme la biotine, la digoxigénine ou la 5-bromo-désoxyuridine. Des marqueurs fluorescents peuvent être également conjugués aux anticorps monoclonaux ou leurs fragments de l'invention et incluent notamment la fluorescéine et ses dérivés, la rhodamine et ses dérivés, la GFP (GFP pour « Green Fluorescent Protein »), le dansyl, l'umbelliférone, etc.. Dans de tels conjugués, les anticorps monoclonaux de l'invention ou leurs fragments peuvent être préparés par des méthodes connues de l'homme de l'art. Ils peuvent être couplés aux enzymes ou aux marqueurs fluorescents directement ou par l'intermédiaire d'un groupe espaceur ou d'un groupe de liaisons tel qu'un polyaldéhyde, comme le glutaraldéhyde, l'acide éthylènediaminetétraacétique (EDTA), l'acide diéthylènetriaminepentaacétique (DPTA), ou en présence d'agents de couplage tels que le périodate, etc.. Les conjugués comportant des marqueurs de type fluorescéine peuvent être préparés par réaction avec un isothiocyanate.

D'autres conjugués peuvent inclure également des marqueurs chimioluminescents tels que le luminol et les dioxétanes ou des marqueurs bioluminescents tels que la luciférase et la luciférine.

Parmi les marqueurs pouvant être fixés sur l'anticorps monoclonal ou un de ses fragments selon l'invention, on préfère également les marqueurs radioactifs tels que ¹⁴C, ³⁶Cl, ⁵⁷Co, ⁵⁸Co, ⁵¹Cr, ¹⁵²Eu, ⁵⁹Fe, ³H, ¹²⁵I,¹³¹I, ³²P, ³³P, ³⁵S, ⁷⁵SE, et ^{99m}Tc qui peuvent être détectés par des moyens connus tels que par exemple le compteur gamma ou à scintillations ou par autoradiographie.

Les peptides et/ou les anticorps selon l'invention, ou une séquence d'acide nucléique codant pour un tel peptide, peuvent, selon un mode de mise en oeuvre de l'invention, entrer dans la composition d'un kit de diagnostic.

Les peptides et les anticorps selon l'invention peuvent être utilisés à titre de médicament, et plus particulièrement pour la préparation d'une composition destinée au traitement préventif ou curatif des affections provoquées par le VRS, sous-groupe A ou B.

Ainsi, l'invention concerne encore l'utilisation d'un peptide selon l'invention ou d'une séquence d'acide nucléique codant pour un tel peptide, ou d'un anticorps selon l'invention pour la préparation d'une composition pharmaceutique, préférentiellement d'un vaccin, destiné au traitement prophylactique ou thérapeutique des affections provoquées par le VRS, sous groupe A ou B présentant une réponse immunogénique, une protection croisée VRS A et B une HSI négative et n'induisant pas d'immunopathologies.

Il est également décrit l'utilisation d'un peptide selon l'invention tel que défini ci-dessus, ou d'une séquence d'acide nucléique codant pour un tel peptide, pour la préparation d'une composition pharmaceutique destinée à générer ou accroître une réponse immunitaire contre le VRS et n'induisant pas d'immunopathologies.

Les légendes des figures et exemples qui suivent sont destinés à illustrer l'invention sans aucunement en limiter la portée.

Dans ces exemples, on se référera aux figures suivantes:
Figures 1A et AB : Titre anti-VRS avant et après une immunisation chez des souris naïves (exprimé en log10).
Figures 2A et 2B : Titre anti-VRS avant et après une immunisation chez des souris séropositives vis-à-vis de VRS-A (exprimé en log10).
Figures 3A et 3B : Titre anti-VRS avant et après une immunisation chez des souris séropositives vis-à-vis de VRS-B (exprimé en log10).
Figures 4A et 4B : protection contre un challenge VRS-A chez des souris immunisées par G23 ou G20.
Figures 5A et 5B : Mesure d'infiltrations de cellules de type granuleux (suivies par le marqueur RB6-8C5) et mesure des cytokines de type Th2 (IL-10 et IL-5).

### Exemple 1 : Production du peptide G20a

A titre d'exemple, le gène codant pour G20a, obtenu par PCR, a été cloné dans un vecteur d'expression dont le promoteur est basé sur l'opéron Tryptophan (Trp). Il en résulte le vecteur nommé pTEXG20a dont l'ADN de l'insert a été vérifié par séquençage ADN. Le vecteur a été transformé dans une bactérie *Escherishia coli* K12 nommée ICONE^{®}.
**A. Fermentation :** dans un fermenteur de 30 1 (CHEMAP CMF400) contenant 18 1 de milieu minimum de culture (g/l) (KH₂PO₄, 6 / K₂HPO₄, 4 / Na₃ citrate 2H₂O, 9/ Extrait de levure 1 / (NH4)₂SO₄, 5 / CaCl₂, 0,3 / MgSO₄, 7H₂O, 2 / Glycérol 100) et les Oligo-éléments (1 ml/l) et de l'antimousse Struktol (0,4 ml/l) supplémenté par une solution de Tétracycline et du Tryptophane à concentration finale respectivement (0,008 g/l) et (0,3 g/l), on inocule 1400 ml du même milieu issu d'une préculture de E. coli recombinante décrite ci-dessus dans un fermenteur de 2 litres. En culture type batch, ces paramètres physico-chimiques sont maintenus constants : Température à 37°C, pH à 7 régulé avec NH₄OH, Agitation 500 - 1 000 tr/mn pour maintenir le taux d'O₂ dissous à 30 %. Lorsque la densité optique (DO 620 nm) du milieu de culture atteint environ la valeur de 50, on peut alors induire l'expression de la protéine recombinante en ajoutant 2 ml/litre de culture d'une solution d'acide 3 indolacrylique (IAA) à 12,5 g/l. Quelques heures après, la fermentation est arrêtée par refroidissement à 4°C après épuisement de substrat carboné (mesuré par un dosage enzymatique du glycérol dans le milieu de culture). La biomasse bactérienne est obtenue par centrifugation continue du milieu (14 000 tr/mn, débit 100 1/h). Le rendement en biomasse est d'environ 38 g de cellules sèches/l.
**B. Extraction :** la biomasse (environ 500 g de cellules sèches) est reprise dans 10 1 de tampon TST (Tris HCl 25 mM pH 8, MgCl₂ 6H₂O 5 mM, EDTA 2mM). La suspension bactérienne est broyée au Manton-Gaulin (3 cycles à 560 bar). La protéine recombinante G20a étant majoritairement soluble, la purification peut être réalisée directement à partir de la suspension broyée. On peut réaliser par exemple la capture de G20a par chromatographie d'échange d'ions en lit expansé (Streamline, Pharmacia). Deux ou trois étapes de chromatographie supplémentaires (échange d'ions et exclusion) sont nécessaires afin d'éliminer les contaminants ADN et protéiques de la cellule hôte. Les protéines purifiées sont analysées sur gel SDS-PAGE dans des conditions réduites, sur l'appareil MINI PROTEAN Il SYSTEM (BioRads). Elles peuvent être visualisées avec du Coomassie brilliant blue R250.

### Exemple 2 : Comparaison de la production du peptide G20a par rapport à la production du peptide G7a

### A. Synthèse et caractérisation du peptide G7a (33 acides aminés)

Le peptide G7a (dénommé également "G7") est un fragment de la protéine G du VRS-A (158-190) de 33 acides aminés. Il comporte 4 cystéines en position 173, 176, 182 et 186, capables de former 2 ponts disulfures. La séquence du peptide G7 est la suivante (SEQ ID N° 11) :
K₁₅₈PNNDFHFEVFNFVPC₁₇₃SIC₁₇₆SNNPTC₁₈₂WAIC₁₈₆KRIP₁₉₀.

Le peptide est obtenu à l'aide d'un synthétiseur automatique de peptide en phase solide (SPPS) en chimie Fmoc/tBu en partant du côté C vers le côté N-terminal à l'échelle de 0,25 mmole à partir des acides aminés protégés suivants : Fmoc-L-Ala, Fmoc-L-Arg(Pmc), Fmoc-L-Asn(Trt), Fmoc-L-Asp(OtBu), Fmoc-L-Cys(Trt), Fmoc-L-Glu(OtBu), Fmoc-L-His(Trt), Fmoc-L-Ile, Fmoc-L-Lys(Boc), Fmoc-L-Phe, Fmoc-L-Pro, Fmoc-L-Ser(tBu), Fmoc-L-Thr(tBu), Fmoc-L-Trp, Fmoc-L-Val, Fmoc-L-Pro-Résine).

1,750 mg de peptide-résine sont obtenus en fin de synthèse. La moitié de l'échantillon (890 mg) est clivé dans une solution contenant de l'acide trifluoroacétique ainsi qu'un scavenger (1 % de TIS) puis lyophilisé avec un rendement de 50 % de peptide réduit non purifié.

81 mg de peptide brut réduit sont solubilisés dans 64 ml d'eau mélangés avec 16 ml de DMSO (solvant oxydant) à température ambiante pendant 5 jours. L'oxydation du mélange brut aboutit à la formation de 2 formes oxydées numérotées ox1 et ox2 d'après l'ordre d'élution en HPLC ("High Pressure Liqui Chromatography") et distinctes de la forme réduite par HPLC. La nature oxydée des 2 formes est confirmée par spectrométrie de masse (4 RSH => 2 RS-S-R moins 4 unités de masse atomique ; masse du peptide réduit : 3842,43 Da ; masse du peptide oxydé : 3838,43 Da).

Les 2 pics principaux observés par RP-HPLC (Reverse Phase-High Pressure >Liquid Chromatography) dans le mélange complexe ainsi obtenu sont isolés et analysés par spectrométrie de masses (ES-MS, "ElectronSpray-Mass Spectrometry"). Les masses mesurées obtenues sont compatibles avec des masses théoriques correspondant à des formes oxydées du peptide G7a (G7ox1 : 1,3 mg soit 1,6 % de rendement) ; RP-HPLC (RT) : 13,70 min. ; ES-MS : masse calculée = 3838,43 Da / Masse mesurée : 3838,27 Da ± 0.10 et G7ox2 : 1,2 mg soit 1,5 % de rendement) ; RP-HPLC (RT) : 15,00 min ; ES-MS : masse calculée = 3838,43 Da / Masse mesurée: 3838,27 Da ± 0.10).

Quatre cystéines (numérotées de 1 à 4 du côté N- vers le côté C-teminal) dans une protéine peuvent s'apparier selon 3 isomères théoriques : 1-2/3-4, 1-3/2-4 et 1-4/2-3. Des méthodes chimiques d'obtention et de caractérisation des 3 isomères théoriques de l'hexadécapeptide G4a, correspondant à la région centrale 172-187 de la protéine G du VRS-A ont été décrites (Beck et al., J. Pept. Res., 55:24, 2000). L'appariement natif de la protéine G est la forme 1-4/2-3 pour le VRS bovin (Langedijk et al., J. Gen. Virol., 77:1249, 1996) et pour le VRS humain (Beck et al., J. Pept. Res., 55:24, 2000). L'appariement des 4 cystéines des 2 formes oxydées du peptide G7a est étudié par LC-MS ("Liquid Chromatography-Mass Spectrometry") et par microséquençage de fragments obtenus suite à une coupure à la thermolysine. L'interprétation des fragments obtenus est décrite dans le tableau 1 ci-après.

**Tableau 1 : Carte peptidique (thermolysine) du peptide purifié G7a-ox2.**

| **Pic UV** | **Masse mesurée** | **Masse théorique** | **Interprétation** | **Attribution** |
|---|---|---|---|---|
| 4 | 247,5 (MH+) | 246,3 | | |
| | 280,5 (MH+) | 279,3 | | |
| | 379,6 (MH+) | 378,3 | | |
| 6 | 849,6 (MH+) | 848,9 | | 2-3 |
| 7 | 276,5 (MH+) | 275,3 | WA | |
| 12 | 1048,23 ± 0,65 | 1841,1 | | 2-4 |
| | 1373,2 | 1373,6 | | 2-4 |
| | 1475,8 | 1474,8 | | 2-4 |
| 14 | 1279,28 ± 0,46 | 1278,6 | | 1-4 |
| 16 | 1035,01 ± 0,21 | 1035,2 | | 2-3 |
| 17 | 1106,41 ± 0,83 | 1106,3 | | 2-3 |
| **Conclusion : G7a-ox2 = G7a 1-4/2-3 + G7a 1-3/2-4** | | | | 1-4/2-3 |
| | | | | 1-3/2-4 |

Il apparaît que le peptide G7ox2 est un mélange inséparable par HPLC des peptides G7(1-4/2-3) et G7(1-3/2-4) en proportion inconnue. Le rendement de la réaction et de la purification est très faible (1,5 %).

### B. Synthèse et caractérisation du peptide G20a (69 acides aminés)

Le peptide G20a est un fragment de la protéine G du VRS-A (140-190)-(144-158) de 69 acides aminés. Il comporte 4 cystéines capables de former 2 ponts disulfures. La séquence du peptide G20a est la suivante (SEQ ID N° 1) :
MEFQ₁₄₀TQPSKPTTKQRQNKPPNKPNNDFHFEVFNFVPC₁₇₃SIC₁₇₆SNNPT C₁₈₂WAIC₁₈₆KRIP₁₉₀S₁₄₄KPTTKQRQNKPPNK₁₅₈.

Le peptide G20a est obtenu par synthèse automatique en phase solide en chimie Fmoc/tBu à l'échelle de 0,25 mmole à partir d'une résine hydroxyméthylphénoxyméthyle (HMP) préchargée avec une Lys (Boc) (0,70 mmole/g) et des Fmoc-acides aminés protégés au niveau des chaînes latérales par les groupes suivants : trityl (Trt) pour Asn, Gln et His ; tert-butyl éther (tBu) pour Ser et Thr ; tert-butyl ester (OtBu) pour Asp et Glu, tert-butyloxycarbonyl (Boc) pour Lys et Trp et 2,2,5,7,8-pentaméthylchromane-6 sulfonyl (Pmc) pour Arg. Les cystéines utilisées possédaient les groupes protecteurs orthogonaux suivants : Trt pour les Cys176 et 182 d'une part et acétamidométhyl (Acm) pour les Cys 173 et 186. A la fin de la synthèse, 1 000 mg des 2 500 mg de peptide-résine ont été clivés par un mélange TFA / EDT / thioanisol / phénol / TIS/ H₂O : 20 ml /0,25 ml / 1 ml / 1,5 g / 0,22 ml / 1ml. Après 3 heures de réaction sous agitation à température ambiante, le mélange est filtré pour éliminer la résine et le peptide brut est précipité grâce à l'addition de diéthyl éther froid. Le précipité est solubilisé dans un mélange H₂O / CH₃CN / TFA : 80 / 20 / 0,1 : v / v / v puis lyophilisé.

Avant oxydation, le peptide brut est purifié par RP-HPLC à l'aide d'un gradient eau / acétonitrile et analysé par RP-HPLC (pureté RP-HPLC > 75 % ; rendement : 38 %) et ES-MS (masse calculée : 8186,42 Da / masse mesurée : 8186,40).

*Formation des ponts disulfures en 2 étapes.* Pour former le pont entre les Cys 176 et 182, non protégées, le peptide lyophilisé est solubilisé (1 mg/ml) dans un mélange DMSO-H₂O à 20 % (v/v) et agité à température ambiante pendant 4 jours (Tam et al, J. Am. Chem. Soc., 113:6657, 1991). En fin de réaction pour éliminer le DMSO, le peptide est purifié par RP-HPLC dans les mêmes conditions que le peptide réduit. Les fractions correspondant au pic principal sont collectées et lyophilisées. Un aliquot est soumis à une analyse par ES-MS pour vérifier que le premier pont disulfure a bien été formé. Le second pont, entre les Cys(Acm) 173 et 186 est obtenu par oxydation à (Buku et al., Int. J. Peptide. Res., 33, 86, 1989 et Annis et al., Meth. Enzymol., 289, 198, 1997). Le peptide est solubilisé (1 mg/ml) dans un mélange acide acétique / eau à 80 % (v/v) and 10 % d'HCl 1 N sont ajoutés. La solution est saturée par de l'azote. Puis 10 équivalents d'iode solubilisé dans un mélange d'acide acétique / eau à 80 % (v/v) sont ajoutés rapidement et le milieu est agité pendant 5 heures à température ambiante. L'excès d'iode est réduit par l'addition goutte à goutte d'une solution aqueuse d'acide ascorbique jusqu'à ce que la couleur caractéristique de l'iode disparaisse. Le peptide oxydé brut est purifié par RP-HPLC, lyophilisé et analysé par RP-HPLC et ES-MS.

*Formation des ponts disulfures en 1 étape.* Un protocole d'obtention en une étape a également par oxydation directe à l'iode sur le peptide réduit permettant également d'obtenir le peptide d'intérêt. Le rendement passe alors de 22 à 44 % (pureté RP-HPLC > 90 % ; masse calculée : 8140,22 Da / masse mesurée : 8040,30 Da).

L'appariement des ponts disulfures est étudié par LC-MS et par microséquençage des fragments obtenus suite à une coupure du peptide à la thermolysine. Les fragments obtenus et leur interprétation sont décrits dans le tableau 2 ci-après.

**Tableau 2 : Carte peptidique (thermolysine) du peptide G20a.**

| **Pic UV** | **Masse mesurée** | **Masse théorique** | **Interprétation** | **Attribution** |
|---|---|---|---|---|
| 29 | 1691,52 ± 0,39 | 1692,1 | | 1-4 |
| 30 | 1591 ± 0,27 | 1591,0 | | 1-4 |
| 34 | 1035,49 ± 0,66 | 1035,2 | | 2-3 |
| 35 | 1106,27 ± 0,33 | 1106,3 | | 2-3 |
| **Conclusion : G20a 1-4/2-3** | | | | 1-4/2-3 |

Il apparaît de façon surprenante que le protocole décrit ci-dessus permet d'obtenir uniquement la forme native G20a (1-4/2-3).

Ainsi, le peptide G20a (69 aa) a été plus facile à produire que le peptide G7a (33 aa) malgré le fait d'additionner 36 aa de plus étape par étape. Le protocole en 2 étapes décrit pour le peptide G20a appliqué au peptide G7a (2 % de rendement) confirmant les résultats reportés dans l'exemple 1. L'explication proposée a posteriori, qui n'est qu'une hypothèse et se veut non limitative, est qu'il est nécessaire d'avoir plus de 4 acides aminés du côté C-terminal de la Cys 186 pour que l'appariement natif entre les Cys 173 et 186 d'une part et les Cys 176 et 182 d'autre part, puisse se faire avec un bon rendement et former un motif structural connu sous le nom de « cystine noose » présent dans la protéine G native (Doreleijers et al., Biochemistry, 35:14684, 1996) et qui correspond à un épitope immunodominant de la protéine G du VRS (Plotnicky et al., J. Virol., 73:5637, 1999).

Cet exemple démontre donc que l'addition d'un fragment peptidique de séquence non sauvage du côté C-terminal de la région conservée des 2 ponts disulfures permet d'en faciliter la synthèse, tout en conservant le motif structural « cystine noose » présent dans la protéine G native.

### Exemple 3 : Immunogénicité et protection

### A. Titre anti-VRS avant et après une immunisation chez des souris naïves

Afin de tester l'immunogénicité des peptides G23a et G20a, les souris Balb/c ont été immunisées avec 6 ou 1,5 µg d'équivalent G2Na deux fois à J0 et J14 par voie intramusculaire.

Le peptide G2Na (dénommé également "G2A" ou "G2a") est le fragment aa 130 - 230 de la protéine G du VRS sous groupe A tel qu'identifié par exemple dans la liste des séquences de la demande WO 95/27787 par la séquence SEQ ID N° 1.

Certaines souris ont été sensibilisées par VRS 20 jours avant la première immunisation afin de les rendre séropositives vis-à-vis de VRS. Les souris ont été ponctionnées avant chaque immunisation et les titres anticorps anti-G2Na, VRS-A et VRS-B ont été déterminés.

Dix jours après la dernière immunisation, les souris ont été challengées avec 10⁵pfu/50µl de VRS-A. Le titre viral a été mesuré 5 jours après le challenge.

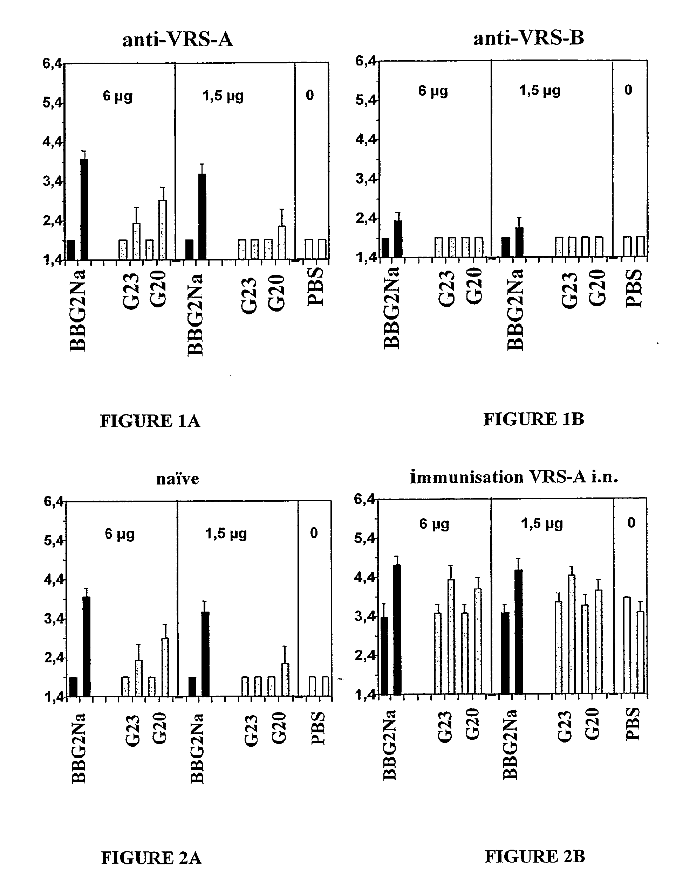

Les souris immunisées par G20a ou G23a développent des réponses anticorps anti-VRS-A et VRS-B dès la première immunisation (voir figures 1A et 1B). Une augmentation est observée après un boost par les différentes molécules testées (résultats non montrés).

### B. Titre anti-VRS avant et après une immunisation chez des souris séropositives vis-à-vis de VRS-A ou de VRS-B (exprimé en log10)

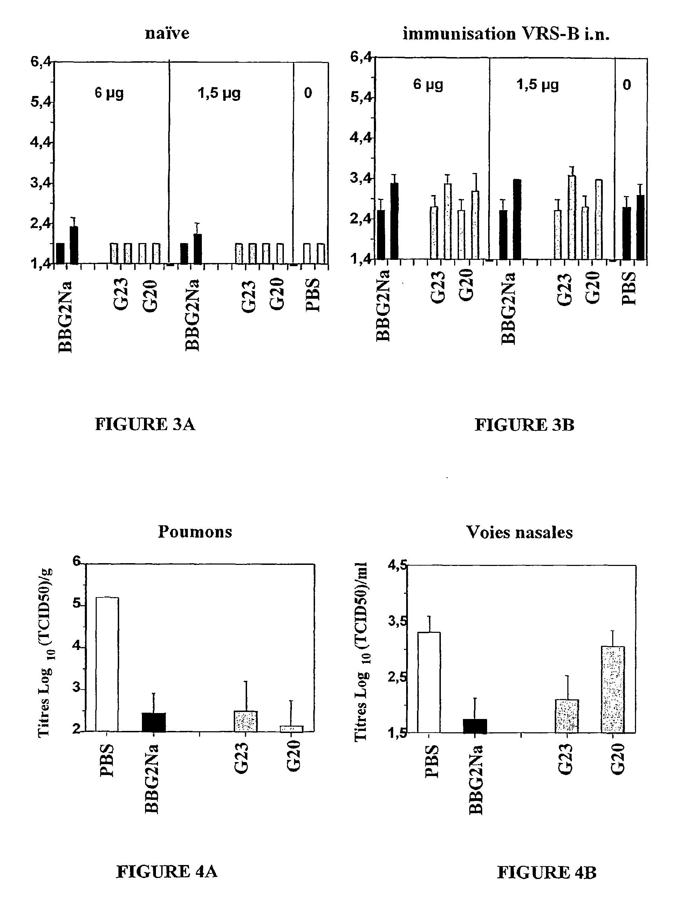

Il a été constaté que les molécules restent immunogéniques même en présence d'anticorps VRS de type A (voir figures 2A et 2B) ou B (voir figures 3A et 3B).

### C. Protection contre un challenge VRS-A chez des souris immunisées par G23a ou G20a

L'immunisation avec G23a ou G20a induit une protection des voies pulmonaires suite à un challenge avec VRS-A (voir figures 4A et 4B).

Les résultats indiquent que G23a et G20a sont immunogéniques et protecteurs chez la souris naïve. De plus, l'immunogénicité et la protection observée avec ces deux molécules sont comparables à celles observées avec BBG2Na (le peptide BBG2Na est le peptide résultant de la fusion du peptide G2Na avec le fragment "BB" de la protéine de liaison à la sérum albumine humaine de Streptocoque, tel qu'identifié dans la demande WO 95/27787).

### Exemple 4 : Détermination du HSI

Les cobayes sont immunisés à J0 et J8 avec les différentes molécules adjuvantées par de l'adjuphos 20 % (v/v) en i.m.. A J21, un rappel est effectué avec les différentes molécules non adjuvantées en i.v. La mort des cobayes est alors évaluée.

Un témoin positif d'expérience constitué d'ovalbumine à 200 µg est inclus pour chaque molécule testée (cf. tableau 3 ci-après).

**Tableau 3**

| | T+ (OVA) | T- | 4 mg | 40 mg |
|---|---|---|---|---|
| G20a | 5/6 | 0/6 | 0/6 | 0/6 |

Les résultats indiquent que G20 n'induit pas de HSI chez 6/6 animaux testés.

### Exemple 5 : Immunopathologies

Les souris sont immunisées trois fois avec G23a adjuvanté par de l'alhydrogel 20 % à J0, J14 et J28. Les souris sont challengées à J34 avec 10⁵ pfu / 50 µl de VRS-A. Sept jours après le challenge, les poumons sont récupérés, digérés et les cellules infiltrant les poumons analysées par FACS ("Fluorescens Activated Cell Sorter"). Les cytokines sont elles aussi analysées par FACS après incubation une nuit avec un activateur non spécifique. L'IL-10 et l'IL-5 sont mesurés.

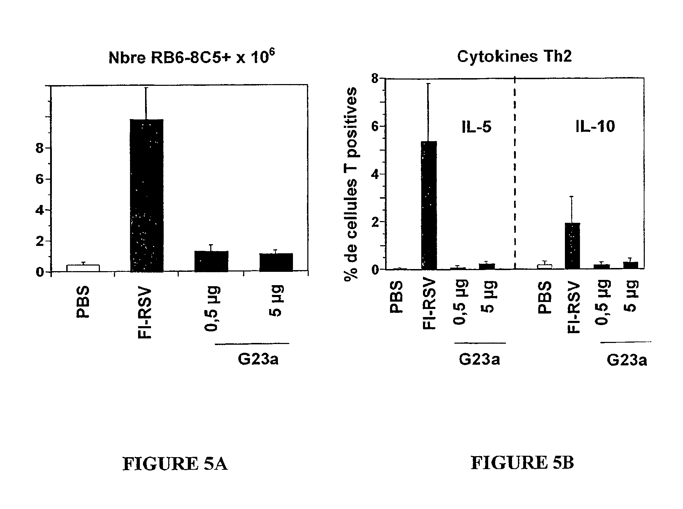

Les résultats (voir figures 5A et 5B) indiquent que G23a, quelle que soit la dose testée, n'induit pas d'infiltrations de cellules de type granuleux (suivies par le marqueur RB6-8C5). En revanche, FI-VRS (VRS inactivé à la formaline), qui induit une immunopathologie, induit une infiltration de ce type de cellules dans les poumons de souris immunisées par FI-VRS et challengées par VRS.

La mesure des cytokines de type Th2 (IL-10 et IL-5) indique que contrairement à FI-VRS, aucune pathologie n'est observée après immunisation par G23a.

## Revendications

1. Peptide immunogène dérivé de la protéine G du VRS (virus syncytial respiratoire) du sous-groupe A ou B comprenant au moins :
- un premier peptide dérivé de la protéine G du VRS du sous-groupe A ou B comprenant au moins en position 173, 176, 182 et 186 une cystéine, et en position C-terminale au plus l'acide aminé en position 192 ; et
- un deuxième peptide dérivé d'une protéine du VRS du sous-groupe A ou B, ledit deuxième peptide étant situé en aval dudit premier peptide, de manière à ce que le peptide immunogène produit présente un pont disulfure reliant les résidus 173 et 186 et un deuxième pont disulfure reliant les résidus 176 et 182, **caractérisé en ce que** ledit peptide immunogène dérivé de la protéine G du VRS du sous-groupe A ou B présente la séquence SEQ ID N° 1, SEQ ID N° 2 ou SEQ ID N° 3.

2. Séquence d'acide nucléique codant pour un peptide immunogène selon la revendication 1.

3. Composition pharmaceutique **caractérisée en ce qu'**elle contient dans un milieu pharmaceutiquement acceptable, au moins un peptide immunogène selon la revendication 1 et/ou une séquence d'acide nucléique selon la revendication 2.

4. Composition selon la revendication 3, **caractérisée en ce qu'**elle contient en outre au moins une protéine porteuse et/ou un adjuvant.

5. Composition selon la revendication 4, **caractérisée en ce que** la protéine porteuse consiste en des dérivés de la protéine DT (toxoïde diphtérique) dans lesquels au moins un résidu cystéine a été supprimé.

6. Composition selon la revendication 5, **caractérisée en ce que** la protéine porteuse consiste en une protéine de séquences SEQ ID N° 4, SEQ ID N° 5 et SEQ ID N° 6.

7. Composition selon la revendication 4, **caractérisée en ce que** l'adjuvant est choisi dans le groupe d'adjuvant comprenant le MPL-A (monophosphoryl lipide A), le MF59^{®}, le Quil-A^{®}, l'ISCOM (« immunostimulating complex »), le Diméthyl Dioctadécyl Ammonium sous forme de bromure (DDAB) ou de chlorure (DDAC), l'alum, l'adjuphos, les CpG, la Leif (facteur d'initiation d'élongation de Leishmania), la CT (toxine cholérique), la LT (« heat Labil Loxin ») et les versions détoxifiées de la CT ou la LT.

8. Composition selon l'une des revendications 3 à 7, **caractérisée en ce que** ledit peptide immunogène est associé, par mélange ou par couplage, à la protéine porteuse et/ou à l'adjuvant.

9. Composition selon l'une des revendications 3 à 8, **caractérisée en ce que** ladite composition pharmaceutique contient en outre un deuxième antigène, immunogène ou haptène du VRS et/ou un antigène, immunogène ou haptène dérivé de micro-organisme responsable de pathologies des voies aériennes choisi parmi les para influenza virus (VIP 1, 2, 3, 4), l'influenza virus (A et B), les hantavirus, les streptocoques, les pneumocoques, haemophilus influenza type b, les rhinovirus, les coronovirus et les méningocoques.

10. Composition selon l'une des revendications 3 à 9, **caractérisée en ce que** ledit milieu pharmaceutiquement acceptable est constitué d'eau, d'une solution aqueuse saline ou d'une solution aqueuse à base de dextrose et/ou de glycérol.

11. Composition selon l'une des revendications 3 à 10, **caractérisée en ce que** ladite composition pharmaceutique est véhiculée sous une forme permettant d'améliorer sa stabilité et/ou son immunogénicité.

12. Anticorps polyclonaux ou monoclonaux dirigés contre un peptide immunogène selon la revendication 1.

13. Anticorps selon la revendication 12, **caractérisés en ce qu'**ils sont humanisés.

14. Kit de diagnostic **caractérisé en ce qu'**il comprend un peptide immunogène selon la revendication 1, une séquence d'acide nucléique selon la revendication 2 ou un anticorps selon la revendication 12 ou 13.

15. Utilisation d'un peptide immunogène selon la revendication 1, d'une séquence d'acide nucléique selon la revendication 2 ou d'un anticorps selon la revendication 12 ou 13 pour la préparation d'une composition pharmaceutique destinée au traitement prophylactique ou thérapeutique des affections provoquées par le VRS, sous groupe A ou B présentant une réponse immunogénique, une protection croisée VRS A et B, une hypersensibilité immédiate négative et n'induisant pas d'immunopathologies.

16. Utilisation d'un peptide immunogène selon la revendication 1, d'une séquence d'acide nucléique selon la revendication 2 ou d'un anticorps selon la revendication 12 ou 13 pour la préparation d'une composition pharmaceutique destinée à générer ou accroître une réponse immunitaire contre le VRS.

17. Polypeptide selon la revendication 1, comprenant aussi une toxine diphtérique, ledit polypeptide étant **caractérisé par** une des séquences SEQ ID N° 7, SEQ ID N° 8 et SEQ ID N° 9.

18. Acide nucléique codant pour un polypeptide selon la revendication 17.

## Claims

1. Immunogenic peptide derived from the G protein of RSV (respiratory syncytial virus) subgroup A or B comprising at least:
- a first peptide derived from the G protein of RSV subgroups A or B comprising at least at position 173, 176, 182 and 186 a cysteine, and the C-terminal end of which comprises at most the amino acid at position 192; and
- a second peptide derived from a protein of RSV subgroup A or B, said second peptide being located downstream of said first peptide, such that the immunogenic peptide produced exhibits a disulfide bridge connecting residues 173 and 186 and a second disulfide bridge connecting residues 176 and 182, **characterized in that** said immunogenic peptide derived from the G protein of RSV subgroup A or B exhibits the sequence SEQ ID No.1, SEQ ID No.2 or SEQ ID No.3.

2. Nucleic acid sequence encoding an immunogenic peptide according to Claim 1.

3. Pharmaceutical composition, **characterized in that** it contains, in a pharmaceutically acceptable medium, at least one immunogenic peptide according to Claim 1 and/or a nucleic acid sequence according to Claim 2.

4. Composition according to Claim 3, **characterized in that** it also contains at least one carrier protein and/or an adjuvant.

5. Composition according to Claim 4, **characterized in that** the carrier protein consists of derivatives of the DT (diphtheria toxoid) protein in which at least one cysteine residue has been deleted.

6. Composition according to Claim 5, **characterized in that** the carrier protein consists of a protein of sequences SEQ ID No.4, SEQ ID No.5 and SEQ ID No.6.

7. Composition according to Claim 4, **characterized in that** the adjuvant is chosen from the adjuvant group comprising MPL-A (monophosphoryl lipid A), MF59^{®}, Quil-A^{®}, ISCOM (immunostimulating complex), dimethyldioctadecylammonium bromide (DDAB) or dimethyldioctadecylammonium chloride (DDAC), alumina, adjuphos, CpGs, Leif (Leishmania elongation initiation factor), CT (cholera toxin), LT (heat labile toxin) and detoxified versions of CT or LT.

8. Composition according to one of Claims 3 to 7, **characterized in that** said immunogenic peptide is associated, by mixing or by coupling, with the carrier protein and/or with the adjuvant.

9. Composition according to one of Claims 3 to 8, **characterized in that** said pharmaceutical composition also contains a second antigen, immunogen or hapten of RSV and/or an antigen, immunogen or hapten derived from a microorganism responsible for pathologies of the airways, chosen from parainfluenza viruses (PIV 1, 2, 3 and 4), influenza virus (A and B), hantaviruses, streptococci, pneumococci, hemophilus influenza type b, rhinoviruses, coronaviruses and meningococci.

10. Composition according to one of Claims 3 to 9, **characterized in that** said pharmaceutically acceptable medium consists of water, of a saline aqueous solution or of an aqueous solution based on dextrose and/or on glycerol.

11. Composition according to one of Claims 3 to 10, **characterized in that** said pharmaceutical composition is vehiculed in a form which makes it possible to improve its stability and/or its immunogenicity.

12. Polyclonal or monoclonal antibody directed against an immunogenic peptide according to Claim 1.

13. Antibody according to Claim 12, **characterized in that** it is humanized.

14. Diagnostic kit, **characterized in that** it comprises an immunogenic peptide according to Claim 1, a nucleic acid sequence according to Claim 2 or an antibody according to Claim 12 or 13.

15. Use of an immunogenic peptide according to Claim 1, of a nucleic acid sequence according to Claim 2 or of an antibody according to Claim 12 or 13, for preparing a pharmaceutical composition intended for the prophylactic or therapeutic treatment of conditions caused by RSV, subgroup A or B, which exhibits an immunogenic response, RSV A and B cross protection and negative immediate hypersensitivity and which does not induce immunopathologies.

16. Use of an immunogenic peptide according to Claim 1, of a nucleic acid sequence according to Claim 2 or of an antibody according to Claim 12 or 13, for preparing a pharmaceutical composition intended to generate or increase an immune response against RSV.

17. Polypeptide according to Claim 1, also comprising a diphtheria toxin, said polypeptide being **characterized by** one of the sequences SEQ ID No.7, SEQ ID No.8 and SEQ ID No.9.

18. Nucleic acid encoding a polypeptide according to Claim 17.

## Patentansprüche

1. Immunogenes Peptid, das vom Protein G des RSV (Respiratory-Syncytial-Virus) der Untergruppe A oder B abgeleitet ist, und mindestens umfasst:
- ein erstes Peptid, das vom Protein G des RSV der Untergruppe A oder B abgeleitet ist und mindestens ein Cystein in der Position 173, 176, 182 und 186 und in der C-terminalen Position höchstens die Aminosäure in Position 192 umfasst; und
- ein zweites Peptid, das von einem Protein des RSV der Untergruppe A oder B abgeleitet ist, wobei das zweite Peptid stromabwärts vom ersten Peptid auf solche Weise angeordnet ist, dass das immunogene Peptid-Produkt eine Disulfid-Brücke, welche die Reste 173 und 186 verbindet, und eine zweite Disulfid-Brücke aufweist, welche die Reste 176 und 182 verbindet, **dadurch gekennzeichnet, dass** das immunogene Peptid, das vom Protein G des RSV der Untergruppe A oder B abgeleitet ist, die Sequenz SEQ ID NO. 1, SEQ ID NO. 2 oder SEQ ID NO. 3 aufweist.

2. Nukleinsäuresequenz, die ein immunogenes Peptid nach Anspruch 1 codiert.

3. Pharmazeutische Zusammensetzung, **dadurch gekennzeichnet, dass** sie in einem pharmazeutisch annehmbaren Medium mindestens ein immunogenes Peptid nach Anspruch 1 und/oder eine Nukleinsäuresequenz nach Anspruch 2 enthält.

4. Zusammensetzung nach Anspruch 3, **dadurch gekennzeichnet, dass** sie darüber hinaus mindestens ein Träger-Protein und/oder ein Adjuvans enthält.

5. Zusammensetzung nach Anspruch 4, **dadurch gekennzeichnet, dass** das Trägerprotein aus Derivaten des DT (Diphterietoxoid)-Proteins besteht, in denen mindestens ein Cysteinrest weggelassen worden ist.

6. Zusammensetzung nach Anspruch 5, **dadurch gekennzeichnet, dass** das Trägerprotein aus einem Protein der Sequenzen SEQ ID NO. 4, SEQ ID NO. 5 und SEQ ID NO. 6 besteht.

7. Zusammensetzung nach Anspruch 4, **dadurch gekennzeichnet, dass** das Adjuvans ausgewählt ist aus der Adjuvansgruppe, die MPL-A (Monophosphoryllipid A), MF59^{®}, Quil-A^{®}, ISCOM ("immunostimulating complex"), Dimethyldioctadecylammonium in Form des Bromids (DDAB) oder des Chlorids (DDAC), Alaun, Adjuphos, CpGs, Leif (Leishmania-Verlängerungsinitiierungsfaktor), CT (Choleratoxin), LT ("Heat Labile Toxin") und die detoxifizierten Versionen von CT oder LT umfasst.

8. Zusammensetzung nach einem der Ansprüche 3 bis 7, **dadurch gekennzeichnet, dass** das immunogene Peptid durch Mischung oder Kupplung mit dem Trägerprotein und/oder dem Adjuvans assoziiert ist.

9. Zusammensetzung nach einem der Ansprüche 3 bis 8, **dadurch** gekennnzeichnet, dass die pharmazeutische Zusammensetzung darüber hinaus ein zweites Antigen, Immunogen oder Hapten des RSV und/oder ein Antigen, Immunogen oder Hapten enthält, das von einem Mikroorganismus abgeleitet ist, der für Krankheiten der Luftwege verantwortlich ist und ausgewählt ist aus Parainfluenza-Virus (PIV 1, 2, 3, 4), Influenza-Virus (A und B), Hantaviren, Streptokokken, Pneumokokken, Haemophilus influenza Typ b, Rhinoviren, Coronoviren und Meningokokken.

10. Zusammensetzung nach einem der Ansprüche 3 bis 9, **dadurch gekennzeichnet, dass** das pharmazeutisch annehmbare Medium aus Wasser, einer wässrigen Kochsalzlösung oder einer wässrigen Lösung auf der Grundlage von Dextrose und/oder Glycerol besteht.

11. Zusammensetzung nach einem der Ansprüche 3 bis 10, **dadurch gekennzeichnet, dass** die pharmazeutische Zusammensetzung in einer Form mit einem Vehikel versehen ist, welche es gestattet, ihre Stabilität und/oder ihre Immunogenität zu verbessern.

12. Monoklonale oder polyklonale Antikörper, die gegen ein immunogenes Peptid nach Anspruch 1 gerichtet sind.

13. Antikörper nach Anspruch 12, **dadurch gekennzeichnet, dass** sie humanisiert sind.

14. Diagnostisches Kit, **dadurch gekennzeichnet, dass** es ein immunogenes Peptid nach Anspruch 1, eine Nukleinsäuresequenz nach Anspruch 2 oder einen Antikörper nach Anspruch 12 oder 13 umfasst.

15. Verwendung eines immunogenen Peptids nach Anspruch 1, einer Nukleinsäuresequenz nach Anspruch 2 oder eines Antikörpers nach Anspruch 12 oder 13 für die Herstellung einer pharmazeutischen Zusammensetzung, welche zur prophylaktischen oder therapeutischen Behandlung von Krankheiten bestimmt ist, die durch RSV, Untergruppe A oder B, hervorgerufen werden, welche eine immunogene Antwort, einen Kreuzschutz gegen RSV A und B, eine negative sofortige Hypersensibilität aufweist und keine Immunkrankheiten induziert.

16. Verwendung eines immunogenen Peptids nach Anspruch 1, einer Nukleinsäuresequenz nach Anspruch 2 oder eines Antikörpers nach Anspruch 12 oder 13 für die Herstellung einer pharmazeutischen Zusammensetzung, die dazu bestimmt ist, eine Immunantwort gegen RSV zu erzeugen oder zu steigern.

17. Polypeptid nach Anspruch 1, ebenfalls umfassend ein Diphterietoxin, wobei das Polypeptid durch eine der Sequenzen SEQ ID NO. 7, SEQ ID NO. 8 und SEQ ID NO. 9 **gekennzeichnet** ist.

18. Nukleinsäure, die ein Polypeptid nach Anspruch 17 codiert.


### LISTE DE SEQUENCES

<110> Pierre Fabre Medicament
<120> NOUVEAUX PEPTIDES DÉRIVÉS DE LA PROTÉINE G DU VRS ET LEUR UTILISATION DANS UN VACCIN
<130> D19677
<150> FR 0109731
   <151> 2001-07-20
<160> 26
<170> PatentIn Ver. 2.1
<210> 1
   <211> 69
   <212> PRT
   <213> Séquence artificielle
<220>
   <223> Description de la séquence artificielle:Peptide dérivé de la protéine G du VRS dénommé G20a
<400> 1
   Met Glu Phe Gln Thr Gln Pro Ser Lys Pro Thr Thr Lys Gln Arg Gln
     1               5                  10                  15
   Asn Lys Pro Pro Asn Lys Pro Asn Asn Asp Phe His Phe Glu Val Phe
                20                  25                  30
   Asn Phe Val Pro Cys Ser Ile Cys Ser Asn Asn Pro Thr Cys Trp Ala
            35                  40                  45
   Ile Cys Lys Arg Ile Pro Ser Lys Pro Thr Thr Lys Gln Arg Gln Asn
        50                  55                  60
   Lys Pro Pro Asn Lys
    65
<210> 2
   <211> 70
   <212> PRT
   <213> Séquence artificielle
<220>
   <223> Description de la séquence artificielle:Peptide dérivé de la protéine G du VRS dénommé G20aP
<400> 2
   Met Glu Phe Gln Thr Gln Pro Ser Lys Pro Thr Thr Lys Gln Arg Gln
   1               5                   10                  15
   Asn Lys Pro Pro Asn Lys Pro Asn Asn Asp Phe His Phe Glu Val Phe
               20                  25                  30
   Asn Phe Val Pro Cys Ser Ile Cys Ser Asn Asn Pro Thr Cys Trp Ala
           35                  40                  45
   Ile Cys Lys Arg Ile Pro Ser Lys Pro Thr Thr Lys Gln Arg Gln Asn
       50                  55                  60
   Lys Pro Pro Asn Lys Pro
   65                  70
<210> 3
   <211> 71
   <212> PRT
   <213> Séquence artificielle
<220>
   <223> Description de la séquence artificielle:Peptide dérivé de la protéine G du VRS dénommé G23a
<400> 3
   Met Glu Phe Gln Thr Gln Pro Ser Lys Pro Thr Thr Lys Gln Arg Gln
   1               5                   10                  15
   Asn Lys Pro Pro Asn Lys Pro Asn Asn Asp Phe His Phe Glu Val Phe
               20                  25                  30
   Asn Phe Val Pro Cys Ser Ile Cys Ser Asn Asn Pro Thr Cys Trp Ala
           35                  40                  45
   Ile Cys Lys Arg Ile Pro Asn Lys Ser Lys Pro Thr Thr Lys Gln Arg
       50                  55                  60
   Gln Asn Lys Pro Pro Asn Lys
   65                  70
<210> 4
   <211> 185
   <212> PRT
   <213> Séquence artificielle
<220>
   <223> Description de la séquence artificielle:Peptide dénommé Dta dérivé du dérivé atoxique CRM 197 de la toxine diphtérique
<400> 4
   Gly Ala Asp Asp Val Val Asp Ser Ser Lys Ser Phe Val Met Glu Asn
    1               5                  10                  15
   Phe Ser Ser Tyr His Gly Thr Lys Pro Gly Tyr Val Asp Ser Ile Gln
               20                  25                  30
   Lys Gly Ile Gln Lys Pro Lys Ser Gly Thr Gln Gly Asn Tyr Asp Asp
           35                  40                  45
   Asp Trp Lys Glu Phe Tyr Ser Thr Asp Asn Lys Tyr Asp Ala Ala Gly
       50                  55                  60
   Tyr Ser Val Asp Asn Glu Asn Pro Leu Ser Gly Lys Ala Gly Gly Val
   65                  70                  75                  80
   Val Lys Val Thr Tyr Pro Gly Leu Thr Lys Val Leu Ala Leu Lys Val
                   85                  90                  95
   Asp Asn Ala Glu Thr Ile Lys Lys Glu Leu Gly Leu Ser Leu Thr Glu
               100                 105                 110
   Pro Leu Met Glu Gln Val Gly Thr Glu Glu Phe Ile Lys Arg Phe Gly
           115                 120                 125
   Asp Gly Ala Ser Arg Val Val Leu Ser Leu Pro Phe Ala Glu Gly Ser
       130                 135                 140
   Ser Ser Val Glu Tyr Ile Asn Asn Trp Glu Gln Ala Lys Ala Leu Ser
   145                 150                 155                 160
   Val Glu Leu Glu Ile Asn Phe Glu Thr Arg Gly Lys Arg Gly Gln Asp
                   165                 170                 175
   Ala Met Tyr Glu Tyr Met Ala Gln Ala
               180                 185
<210> 5
   <211> 255
   <212> PRT
   <213> Séquence artificielle
<220>
   <223> Description de la séquence artificielle:Peptide dénommé Dtb dérivé du dérivé atoxique CRM 197 de la toxine diphtérique
<400> 5
   Ile Asn Leu Asp Trp Asp Val Ile Arg Asp Lys Thr Lys Thr Lys Ile
   1               5                   10                  15
   Glu Ser Leu Lys Glu His Gly Pro Ile Lys Asn Lys Met Ser Glu Ser
               20                  25                  30
   Pro Asn Lys Thr Val Ser Glu Glu Lys Ala Lys Gln Tyr Leu Glu Glu
           35                  40                  45
   Phe His Gln Thr Ala Leu Glu His Pro Glu Leu Ser Glu Leu Lys Thr
       50                  55                  60
   Val Thr Gly Thr Asn Pro Val Phe Ala Gly Ala Asn Tyr Ala Ala Trp
   65                  70                  75                  80
   Ala Val Asn Val Ala Gln Val Ile Asp Ser Glu Thr Ala Asp Asn Leu
                   85                  90                  95
   Glu Lys Thr Thr Ala Ala Leu Ser Ile Leu Pro Gly Ile Gly Ser Val
               100                 105                 110
   Met Gly Ile Ala Asp Gly Ala Val His His Asn Thr Glu Glu Ile Val
           115                 120                 125
   Ala Gln Ser Ile Ala Leu Ser Ser Leu Met Val Ala Gln Ala Ile Pro
       130                 135                 140
   Leu Val Gly Glu Leu Val Asp Ile Gly Phe Ala Ala Tyr Asn Phe Val
   145                 150                 155                 160
   Glu Ser Ile Ile Asn Leu Phe Gln Val Val His Asn Ser Tyr Asn Arg
                   165                 170                 175
   Pro Ala Tyr Ser Pro Gly His Lys Thr Gln Pro Phe Leu His Asp Gly
               180                 185                 190
   Tyr Ala Val Ser Trp Asn Thr Val Glu Asp Ser Ile Ile Arg Thr Gly
           195                 200                 205
   Phe Gln Gly Glu Ser Gly His Asp Ile Lys Ile Thr Ala Glu Asn Thr
       210                 215                 220
   Pro Leu Pro Ile Ala Gly Val Leu Leu Pro Thr Ile Pro Gly Lys Leu
   225                 230                 235                 240
   Asp Val Asn Lys Ser Lys Thr His Ile Ser Val Asn Gly Arg Lys
                   245                 250                 255
<210> 6
   <211> 440
   <212> PRT
   <213> Séquence artificielle
<220>
   <223> Description de la séquence artificielle:Peptide fusionné dénommé DtaDTb dérivé du dérivé atoxique CRM 197 de la toxine diphtérique
<400> 6
   Gly Ala Asp Asp Val Val Asp Ser Ser Lys Ser Phe Val Met Glu Asn
    1               5                  10                  15
   Phe Ser Ser Tyr His Gly Thr Lys Pro Gly Tyr Val Asp Ser Ile Gln
               20                  25                  30
   Lys Gly Ile Gln Lys Pro Lys Ser Gly Thr Gln Gly Asn Tyr Asp Asp
           35                  40                  45
   Asp Trp Lys Glu Phe Tyr Ser Thr Asp Asn Lys Tyr Asp Ala Ala Gly
       50                  55                  60
   Tyr Ser Val Asp Asn Glu Asn Pro Leu Ser Gly Lys Ala Gly Gly Val
   65                  70                  75                  80
   Val Lys Val Thr Tyr Pro Gly Leu Thr Lys Val Leu Ala Leu Lys Val
                   85                  90                  95
   Asp Asn Ala Glu Thr Ile Lys Lys Glu Leu Gly Leu Ser Leu Thr Glu
               100                 105                 110
   Pro Leu Met Glu Gln Val Gly Thr Glu Glu Phe Ile Lys Arg Phe Gly
           115                 120                 125
   Asp Gly Ala Ser Arg Val Val Leu Ser Leu Pro Phe Ala Glu Gly Ser
       130                 135                 140
   Ser Ser Val Glu Tyr Ile Asn Asn Trp Glu Gln Ala Lys Ala Leu Ser
   145                 150                 155                 160
   Val Glu Leu Glu Ile Asn Phe Glu Thr Arg Gly Lys Arg Gly Gln Asp
                   165                 170                 175
   Ala Met Tyr Glu Tyr Met Ala Gln Ala Ile Asn Leu Asp Trp Asp Val
               180                 185                 190
   Ile Arg Asp Lys Thr Lys Thr Lys Ile Glu Ser Leu Lys Glu His Gly
           195                 200                 205
   Pro Ile Lys Asn Lys Met Ser Glu Ser Pro Asn Lys Thr Val Ser Glu
       210                 215                 220
   Glu Lys Ala Lys Gln Tyr Leu Glu Glu Phe His Gln Thr Ala Leu Glu
   225                 230                 235                 240
   His Pro Glu Leu Ser Glu Leu Lys Thr Val Thr Gly Thr Asn Pro Val
                   245                 250                 255
   Phe Ala Gly Ala Asn Tyr Ala Ala Trp Ala Val Asn Val Ala Gln Val
               260                 265                 270
   Ile Asp Ser Glu Thr Ala Asp Asn Leu Glu Lys Thr Thr Ala Ala Leu
           275                 280                 285
   Ser Ile Leu Pro Gly Ile Gly Ser Val Met Gly Ile Ala Asp Gly Ala
       290                 295                 300
   Val His His Asn Thr Glu Glu Ile Val Ala Gln Ser Ile Ala Leu Ser
   305                 310                 315                 320
   Ser Leu Met Val Ala Gln Ala Ile Pro Leu Val Gly Glu Leu Val Asp
                   325                 330                 335
   Ile Gly Phe Ala Ala Tyr Asn Phe Val Glu Ser Ile Ile Asn Leu Phe
               340                 345                 350
   Gln Val Val His Asn Ser Tyr Asn Arg Pro Ala Tyr Ser Pro Gly His
           355                 360                 365
   Lys Thr Gln Pro Phe Leu His Asp Gly Tyr Ala Val Ser Trp Asn Thr
       370                 375                 380
   Val Glu Asp Ser Ile Ile Arg Thr Gly Phe Gln Gly Glu Ser Gly His
   385                 390                 395                 400
   Asp Ile Lys Ile Thr Ala Glu Asn Thr Pro Leu Pro Ile Ala Gly Val
                   405                 410                 415
   Leu Leu Pro Thr Ile Pro Gly Lys Leu Asp Val Asn Lys Ser Lys Thr
               420                 425                 430
   His Ile Ser Val Asn Gly Arg Lys
           435                 440
<210> 7
   <211> 255
   <212> PRT
   <213> Séquence artificielle
<220>
   <223> Description de la séquence artificielle:Peptide fusionné dénommé G20a-DTa dérivé de la protéine G du VRS et du dérivé atoxique CRM 197 de la toxine diphtérique
<400> 7
   Met Glu Phe Gln Thr Gln Pro Ser Lys Pro Thr Thr Lys Gln Arg Gln
   1               5                   10                  15
   Asn Lys Pro Pro Asn Lys Pro Asn Asn Asp Phe His Phe Glu Val Phe
               20                  25                  30
   Asn Phe Val Pro Cys Ser Ile Cys Ser Asn Asn Pro Thr Cys Trp Ala
           35                  40                  45
   Ile Cys Lys Arg Ile Pro Ser Lys Pro Thr Thr Lys Gln Arg Gln Asn
       50                  55                  60
   Lys Pro Pro Asn Lys Pro Gly Ala Asp Asp Val Val Asp Ser Ser Lys
   65                  70                  75                  80
   Ser Phe Val Met Glu Asn Phe Ser Ser Tyr His Gly Thr Lys Pro Gly
                   85                  90                  95
   Tyr Val Asp Ser Ile Gln Lys Gly Ile Gln Lys Pro Lys Ser Gly Thr
               100                 105                 110
   Gln Gly Asn Tyr Asp Asp Asp Trp Lys Glu Phe Tyr Ser Thr Asp Asn
           115                 120                 125
   Lys Tyr Asp Ala Ala Gly Tyr Ser Val Asp Asn Glu Asn Pro Leu Ser
       130                 135                 140
   Gly Lys Ala Gly Gly Val Val Lys Val Thr Tyr Pro Gly Leu Thr Lys
   145                 150                 155                 160
   Val Leu Ala Leu Lys Val Asp Asn Ala Glu Thr Ile Lys Lys Glu Leu
                   165                 170                 175
   Gly Leu Ser Leu Thr Glu Pro Leu Met Glu Gln Val Gly Thr Glu Glu
               180                 185                 190
   Phe Ile Lys Arg Phe Gly Asp Gly Ala Ser Arg Val Val Leu Ser Leu
           195                 200                 205
   Pro Phe Ala Glu Gly Ser Ser Ser Val Glu Tyr Ile Asn Asn Trp Glu
       210                 215                 220
   Gln Ala Lys Ala Leu Ser Val Glu Leu Glu Ile Asn Phe Glu Thr Arg
   225                 230                 235                 240
   Gly Lys Arg Gly Gln Asp Ala Met Tyr Glu Tyr Met Ala Gln Ala
                   245                 250                 255
<210> 8
   <211> 325
   <212> PRT
   <213> Séquence artificielle
<220>
   <223> Description de la séquence artificielle:Peptide fusionné dénommé G20a-DTb dérivé de la protéine G du VRS et du dérivé atoxique CRM 197 de la toxine diphtérique
<400> 8 <210> 9
   <211> 510
   <212> PRT
   <213> Séquence artificielle
<220>
   <223> Description de la séquence artificielle:Peptide fusionné dénommé G20a-DTaDTb dérivé de la protéine G du VRS et du dérivé atoxique CRM 197 de la toxine diphtérique
<400> 9
<210> 10
   <211> 9
   <212> PRT
   <213> Séquence artificielle
<220>
   <223> Description de la séquence artificielle:Peptide dérivé de la protéine G du VRS
<400> 10 <210> 11
   <211> 33
   <212> PRT
   <213> Séquence artificielle
<220>
   <223> Description de la séquence artificielle:Peptide dérivé de la protéine G du VRS
<400> 11
<210> 12
   <211> 8
   <212> PRT.
   <213> Séquence artificielle
<220>
   <223> Description de la séquence artificielle:Peptide dérivé de la protéine G du VRS
<400> 12
   Ile Cys Ser Asn Asn Pro Thr Cys
     1               5
<210> 13
   <211> 6
   <212> PRT
   <213> Séquence artificielle
<220>
   <223> Description de la séquence artificielle:Peptide dérivé de la protéine G du VRS
<400> 13
   Ile Cys Lys Arg Ile Pro
    1               5
<210> 14
   <211> 6
   <212> PRT
   <213> Séquence artificielle
<220>
   <223> Description de la séquence artificielle:Peptide dérivé de la protéine G du VRS
<400> 14
   Ile Cys Ser Asn Asn Pro
    1               5
<210> 15
   <211> 6
   <212> PRT
   <213> Séquence artificielle
<220>
   <223> Description de la séquence artificielle:Peptide dérivé de la protéine G du VRS
<400> 15
   Ile Cys Lys Arg Ile Pro
    1               5
<210> 16
   <211> 7
   <212> PRT
   <213> Séquence artificielle
<220>
   <223> Description de la séquence artificielle:Peptide dérivé de la protéine G du VRS
<400> 16
   Ile Cys Ser Asn Asn Pro Thr
    1               5
<210> 17
   <211> 6
   <212> PRT
   <213> Séquence artificielle
<220>
   <223> Description de la séquence artificielle:Peptide dérivé de la protéine G du VRS
<400> 17
   Ile Cys Lys Arg Ile Pro
   1 5
<210> 18
   <211> 5
   <212> PRT
   <213> Séquence artificielle
<220>
   <223> Description de la séquence artificielle:Peptide dérivé de la protéine G du VRS
<400> 18
   Phe Val Pro Cys Ser
   1 5
<210> 19
   <211> 6
   <212> PRT
   <213> Séquence artificielle
<220>
   <223> Description de la séquence artificielle:Peptide dérivé de la protéine G du VRS
<400> 19
   Ile Cys Lys Arg Ile Pro
   1 5
<210> 20
   <211> 9
   <212> PRT
   <213> Séquence artificielle
<220>
   <223> Description de la séquence artificielle:Peptide dérivé de la protéine G du VRS
<400> 20
   Ile Cys Ser Asn Asn Pro Thr Cys Trp
   1 5
<210> 21
   <211> 10
   <212> PRT
   <213> Séquence artificielle
<220>
   <223> Description de la séquence artificielle:Peptide dérivé de la protéine G du VRS
<400> 21
   Ile Cys Ser Asn Asn Pro Thr Cys Trp Ala
   1 5 10
<210> 22
   <211> 6
   <212> PRT
   <213> Séquence artificielle
<220>
   <223> Description de la séquence artificielle:Peptide dérivé de la protéine G du VRS
<400> 22
   Phe Val Pro Cys Ser Ile
   1 5
<210> 23
   <211> 9
   <212> PRT
   <213> Séquence artificielle
<220>
   <223> Description de la séquence artificielle:Peptide dérivé de la protéine G du VRS
<400> 23
   Ile Cys Lys Arg Ile Pro Ser Lys Pro
   1 5
<210> 24
   <211> 5
   <212> PRT
   <213> Séquence artificielle
<220>
   <223> Description de la séquence artificielle:Peptide dérivé de la protéine G du VRS
<400> 24
   Phe Val Pro Cys Ser
   1 5
<210> 25
   <211> 9
   <212> PRT
   <213> Séquence artificielle
<220>
   <223> Description de la séquence artificielle:Peptide dérivé de la protéine G du VRS
<400> 25
   Ile Cys Ser Asn Asn Pro Thr Cys Trp
    1               5
<210> 26
   <211> 25
   <212> PRT
   <213> Séquence artificielle
<220>
   <223> Description de la séquence artificielle:Peptide dérivé de la protéine G du VRS
<400> 26
   Phe Val Pro Cys Ser Ile Cys Ser Asn Asn Pro Thr Cys Trp Ala Ile
    1               5                   10                  15
   Cys Lys Arg Ile Pro Ser Lys Pro Thr
                20                  25